(19) Europäisches Patentamt
European Patent Office
Office européen des brevets

(11) Publication number: **0 014 565**
**B1**

(12) # EUROPEAN PATENT SPECIFICATION

(45) Date of publication of patent specification: **23.02.83**

(51) Int. Cl.³: **C 07 D 501/00,**
C 07 D 501/59
//C07D501/12

(21) Application number: **80300281.5**

(22) Date of filing: **31.01.80**

(54) **3-Hydroxycephalosporin solvates and process for their production.**

(30) Priority: **01.02.79 US 8468**

(43) Date of publication of application:
**20.08.80 Bulletin 80/17**

(45) Publication of the grant of the patent:
**23.02.83 Bulletin 83/8**

(84) Designated Contracting States:
**DE GB NL SE**

(56) References cited:
**FR - A - 2 271 830**
**US - A - 4 044 002**

(73) Proprietor: **ELI LILLY AND COMPANY**
**307, East McCarty Street**
**Indianapolis Indiana 46285 (US)**

(72) Inventor: **Fisher, Jack Wayne**
**2014 Woodcrest Road**
**Indianapolis, Indiana (US)**

(74) Representative: **Hudson, Christopher Mark et al,**
**European Patent Attorney Erl Wood Manor**
**Windlesham Surrey GU20 6PH (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 014 565 B1

# 3-Hydroxycephalosporin solvates and process for their production

This invention relates to novel cephalosporin solvates.

3-Hydroxy-3-cephems and the corresponding sulfoxides have been described as intermediates in the chemical conversion of penicillins to cephalosporins substituted directly at C-3 with halo, alkoxy or other functional groups. Tsuji disclosed in U.S. Patent 4,079,181 the preparation of 3-hydroxy-3-cephems from the penicillin sulfoxide derived bicyclic thiazolineazetidinones [U.S. Patent No. 3,705,892]. 3-Hydroxy-3-cephems have also been prepared by ozonolysis of the corresponding 3-exomethylenecephams and penicillin sulfoxide derived 3-exomethylenecepham sulfoxides [U.S. Patent No. 4,052,387] as described by Chauvette in U.S. Patent Nos. 3,917,587 and 4,060,688. Also Hatfield recently described the reduction of 3-exomethylenecepham and 3-hydroxycepham sulfoxides to the corresponding sulfides using acetyl bromide as a reducing agent. The preparation of 3-halocephems and 3-hydroxycephem ethers from 3-hydroxycephems has been disclosed by Chauvette in U.S. Patent Nos. 3,925,372 and 3,917,587 respectively. A clinically significant cephalosporin antibiotic which is prepared from 3-hydroxycephem intermediates is 7-[D-(2-amino-2-phenylacetamido)]-3-chloro-3-cephem-4-carboxylic acid.

This invention is directed to crystalline acetic acid, propionic acid, methylene chloride and methanol solvates of 4'-nitrobenzyl 7-phenoxyacetamido 3-hydroxy-3-cephem-4-carboxylate and the corresponding sulfoxide. The discovery of the present solvates allows for facile isolation and purification of 4'-nitrobenzyl 7-phenoxyacetamido-3-hydroxy-3-cephem-4-carboxylate and its 1-oxide.

The present invention is directed to 4'-nitrobenzyl 7-phenoxyacetamido-3-hydroxy-3-cephem-4-carboxylate solvates of the formula

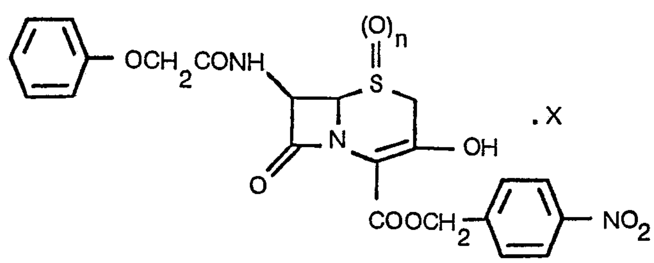

wherein n is 1 or 0; and X is $CH_3COOH$, $CH_3CH_2COOH$ or $CH_3OH$ when n is 1, and X is $CH_3COOH$, $CH_3CH_2COOH$ or 1/2 $CH_2Cl_2$ when n is 0.

The acetic acid solvates, a preferred embodiment of the present invention, contain 1 molar equivalent of acetic acid for each mole of 3-hydroxycephem. They are prepared simply by adding acetic acid to solutions containing 4'-nitrobenzyl 7-phenoxyacetamido-3-hydroxy-3-cephem-4-carboxylate or its 1 oxide. Depending, of course, on the concentration of the 3-hydroxycephem in solution and the particular solvent being used, the acetic acid solvate will crystallize spontaneously or it can be caused to crystallize by using conventional crystallization techniques such as lowering the solution temperature, adding an anti-solvent or seeding with crystals of the desired solvate or by using any combination of these crystallization techniques. The crystalline acetic acid solvates are isolated by filtration.

Solvents from which the acetic acid solvates of the present invention can be crystallized are generally those organic solvents in which the 3-hydroxycephem (sulfoxide) is soluble. Suitable solvents include halogenated hydrocarbons such as methylene chloride, chloroform, ethylene dichloride, and 1,1,2-trichloroethane; cyclic ethers such as tetrahydrofuran and dioxane; and amides such as dimethylformamide or dimethylacetamide. Alternatively the acetic acid solvates can be isolated from acetic acid itself. Anti-solvents for crystallizing the present acetic acid solvates from water immiscible solutions are lower aliphatic hydrocarbons such as pentane, hexane, cyclohexane or "petroleum ethers". Water can be employed advantageously in crystallizing the present acetic acid solvates from water miscible solvents such as dimethylformamide or acetic acid.

The solutions from which the present acetic acid solvates are isolated can be reaction mixtures in which the 3-hydroxycephem (sulfoxide) has been prepared for such solutions can be prepared by simply dissolving the 3-hydroxy-3-cephem (sulfoxide), usually in an impure state, in the desired solvent for the purpose of purification by crystallization as the acetic acid solvate. The acetic acid solvate of 4'-nitrobenzyl 7-phenoxyacetamido-3-hydroxy-3-cephem-3-carboxylate 1-oxide which crystallized from acetic acid/water had a melting point of 138—140°C.

The propionic acid solvates of the present invention are prepared, crystallized and isolated following procedures analogous to those described above for the acetic acid solvates with the exception, of course, of substituting propionic acid for acetic acid. The propionic acid solvate of 4'-nitrobenzyl 7-phenoxyacetamido-3-hydroxy-3-cephem-4-carboxylate which crystallized from a methylene chloride/propionic acid mixture had a melting point of 89—91°C. while the corresponding 3-hydroxycephem sulfoxide propionic acid solvate (from dimethylformamide/water/propionic acid) melted at 149—150°C.

The methylene chloride solvate of the present invention contains, .5 molar equivalents of methylene chloride for each mole of 3-hydroxy-3-cephem ester. It is prepared typically by adding an antisolvent such as hexane to methylene chloride solutions of 4'-nitrobenzyl 7-phenoxyacetamido-3-hydroxy-3-cephem-4-carboxylate to the cloud point with subsequent cooling and optional seeding of the solution.

Characteristic of each of the aforedescribed solvates is the fact that vacuum drying, even at elevated temperatures (30—50°C), fails to destroy the solvate. Heating the solvates to their fusion point will however free the bound solvent.

The methanol solvate of 4'-nitrobenzyl 7-phenoxyacetamido-3-hydroxy-3-cephem-4-carboxylate-1-oxide contains 1 molar equivalent of methanol for each mole of 3-hydroxycephem sulfoxide. It is prepared simply by slurrying a sample of the aforedescribed acetic acid solvate or propionic acid solvate of 4-nitrobenzyl 7-phenoxyacetamido 3-hydroxy-3-cephem-4-carboxylate 1-oxide in methanol for about 2—3 hours at room temperature. This methanol solvate is a "weaker" solvate than the corresponding acetic acid or propionic acid solvates. That, is vacuum drying of the methanol solvate at 30—50°C for about 12 to 24 hours provides an anhydrous, unsolvated sample of the 3-hydroxycephem sulfoxide. Thus, an impure sample of 4'-nitrobenzyl 7-phenoxyacetamido-3-hydroxy-3-cephem-4-carboxylate 1 oxide can be purified by first crystallizing it as its acetic acid or propionic acid solvate, preparing the methanol solvate by slurrying the acid solvate with methanol, and vacuum drying the methanol solvate to the anhydrous, unsolvated 3-hydroxycephem sulfoxide.

The preparation of 4'-nitrobenzyl 7-phenoxyacetamido-3-hydroxy-3-cephem-4-carboxylate and its 1-oxide are described in U.S. Patent Nos. 4,044,002 and 4,060,688 respectively. U.S. Patents 4,079,181, 3,917,587, 3,925,372 and 3,917,588 further describe the preparation of these compounds and their conversion to antibiotic compounds.

The following examples are provided to further illustrate the present invention.

## Example 1

4'-Nitrobenzyl 7-phenoxyacetamido-3-hydroxy-3-cephem-4-carboxylate methylene chloride solvate

To a solution of 150 g (298.7 mmol) of 4'-nitrobenzyl 7-phenoxyacetamido-3-hydroxy-3-cephem-4-carboxylate 1-oxide in 1500 ml of methylene chloride was added 75 ml (705 mmol) of amylene and then 54 ml (729 mmol) of acetyl bromide dropwise over a 10 minute period keeping the temperature at 20—25°C. After 35 minutes at room temperature the reaction mixture was washed with water (2 x 1250 ml) and brine (1250 ml), dried over $Na_2SO_4$, and evaporated *in vacuo* to a volume of 400 ml. Hexane (800 ml) was added dropwise. The mixture was seeded when it turned cloudy during the hexane addition. The product crystallized. After 30 minutes the mixture was filtered. The crystalline solid was washed with 500 ml of 2:1/hexane:methylene chloride and dried at 40° under reduced pressure overnight to provide 135.1 g of the title product: m.p. 75°C (dec.)

nmr (DMSO d—6) $\delta$ 3.56 (ABq, 2), 4.66 (s, 2), 5.24 (d, l, J = 4 Hz), 5.43 (s, 2), 5.45 (q, l, J = 4 and 8 Hz), 5.77 (s, l, 1/2 $CH_2Cl_2$), 7.0—8.4 (ArH) and 9.07 (d, l, J = 8 Hz).

Anal. Calcd. for $C_{22.5}H_{20}N_3O_8SCl$:

C, 51.19; H, 3.82; Cl, 6.72; N, 7.96; O, 24.24; S, 6.07.

Found:

C, 51.15; H, 3.88; Cl, 6.46; N, 8.15; O, 24.20; S, 6.04.

## Example 2

4'-Nitrobenzyl 7-phenoxyacetamido-3-hydroxy-3-cephem-4-carboxylate acetic acid solvate

(A) To a solution of 4'-nitrobenzyl 7-phenoxyacetamido-3-hydroxy-3-cephem-4-carboxylate 1-oxide, derived from ozonolysis of 15 g of 4'-nitrobenzyl 7-phenoxyacetamido-3-methylenecepham-4-carboxylate sulfoxide, in 135 ml of methylene chloride at 15°C was added 8.8 ml of amylene and 6.1 ml of acetyl bromide. After 2 hours of additional 1.86 ml of amylene and 1.3 ml of acetyl bromide were added. After 1 hour and again after 2 hours 25 ml of proplyenecarbonate was added to the reaction mixture to increase the solubility of the 3-hydroxy sulfoxide. The reaction mixture was warmed to 30—35° before the heat source was removed. After 6 hours at room temperature the reaction mixture was washed with water (4 x 500 ml) and then evaporated *in vacuo* to a weight of 55 g. The title product crystallized after 75 ml of acetic acid was added to the reaction mixture. The crystalline product was filtered, washed and dried — 6.93 g. m.p. 116—118°C.

A nuclear magnetic resonance spectrum of the dried product was identical to that of the known unsolvated material except for the presence of 1 equivalent of acetic acid (by nmr integration).

(B) In another experiment the unsolvated 4'-nitrobenzyl 7-phenoxyacetamido-3-hydroxy-3-cephem-4-carboxylate was obtained as a foam from the reduction of 4.0 grams of the corresponding sulfoxide with $PCl_3$. The product was dissolved in 25 ml of acetic acid. The acetic acid solvate (1.44 g) crystallized from the acetic acid solution.

(C) To a solution of the 3-hydroxy cephem sulfoxide (7.52 g, 5 mmol) in 75 ml of methylene chloride was added 4.33 g. (16.5 mmol) of triphenyl phosphine and 2.34 ml (33 mmol) of acetyl

chloride. The mixture was refluxed (41°C) for about 4 hours. The mixture was washed with water (5 × 50 ml) and brine (25 ml), dried over $Na_2SO_4$ and then evaporated *in vacuo* to 22 g. of a syrup. Attempts to crystallize the product failed until solution was treated with 2 ml of acetic acid. The mixture was diluted with 30 ml of 3:1/hexane:methylene chloride and filtered to afford after drying, 6.30 g of the titled acetic acid solvate; m.p. 111—115°C.

Example 3

4'-Nitrobenzyl 7-phenoxyacetamido-3-hydroxy-3-cephem-4-carboxylate 1-oxide acetic acid solvate

Impure 4'-nitrobenzyl 7-phenoxyacetamido-3-hydroxy-3-cephem-4-carboxylate 1-oxide (400 g) was dissolved in 1000 ml of dimethylformamide. After 30 minutes the dark solution was treated with 4 g of Darco and then filtered through Hyflo to remove insolubles. The filtrate was diluted with 1200 ml of acetic acid and seeded. After about 15 minutes the product began to crystallize. Water (1200 ml) was added dropwise. Thirty minutes after water addition was complete the title product was filtered and washed with 1:1/water:acetic acid (500 ml) and water (500 ml). The product was air dried over night a 40°C. Yield — 373.6 g.

nmr (DMSO d—6) $\delta$ 1.97 (s, 3, $CH_3COOH$), 4.09 (bs, 2), 4.78 (bs, 2), 5.12 (d, l, J = 5 Hz), 5.53 (bs, 2), 6.0 (q, l, J = 5 and 10Hz), and 6.9—8.4 (ArH).

Anal. Calcd. for $C_{24}H_{23}N_3O_{11}S$:

C, 51.34; H, 4.13; N, 7.48; O, 5.71.

Found:

C, 50.83; H, 4.12; N, 7.17; O, 5.23.

Example 4

4'-Nitrobenzyl 7-phenoxyacetamido-3-hydroxy-3-cephem-4-carboxylate 1-oxide methanol solvate

(A) To 100 ml of methanol was added 10 g. of 4'-nitrobenzyl 7-phenoxyacetamido-3-hydroxy-3-cephem-4-carboxylate 1-oxide acetic acid solvate. After 3 hours at room temperature the solution was cooled in ice for 30 minutes and filtered. The crystalline product was washed with cold methanol and dried — 7.85 g. The product softened at 87—88°C and then solidified before melting again at 118—120°C. An nmr spectrum showed the product to contain 1 equivalent methanol.

(B) 4'-Nitrobenzyl 7-phenoxyacetamido-3-hydroxy-3-cephem-4-carboxylate 1-oxide acetic acid solvate (derived from ozonolysis of the corresponding 3-methylenecepham sulfoxide im methylene chloride using acetic acid/water to effect crystallization) was suspended in 150 ml of methanol with seeding and stirring at room temperature for one hour. The recrystallized solid appeared finer than the acetic acid solvate crystals. The slurry was cooled in ice for 30 minutes and filtered. After washing the methanol solvate with cold methanol the solvate was dried over night at 40—45°C in a vacuum and then at 50—55°C for 4 hours affording 13.77 g. of a white powder m.p. 105—110°C (clear at 120°C). An nmr spectrum showed the product to be free of solvate (acetic acid or methanol).

Example 5

4'-Nitrobenzyl 7-phenoxyacetamido-3-hydroxy-3-cephem-4-carboxylate propionic acid solvate

A 5.28 g portion of the methylene chloride solvate from Example 1 was dissolved in 25 ml of methylene chloride at room temperature. The resulting solution was diluted with 25 ml of propionic acid. The volume of the mixture was then reduced by evaporation *in vacuo*. Crystals of the title product formed during the evaporation. After 20 minutes the product was isolated by filtration, washed with 15 ml of propionic acid and dried in vacuo at 35°C. A total of 5.09 g of the title product was isolated.

nmr ($CDCl_3$) $\delta$ 1.16 (t, 3, $CH_3CH_2COOH$), 2.4 (q, 2, $CH_3CH_2COOH$) 3.45 (bs, 2, $C_2$—H), 2.96 (s, 2, $C_6H_3OCH_2$) 5.13 (d, l, J = 4 Hz, $C_6$—H), 5.45 (ABq, 2, ester $CH_2$), 5.75 (q, l, J = 4 Hz and 8 Hz, $C_7$—H), and 6.9—8.4 (m, 10, NH + ArH).

Anal. Calcd. for $C_{25}H_{25}N_3O_{10}S$:

C, 53.66; H, 4.50; N, 7.51; S, 5.73.

Found:

C, 53.37; H, 4.41; N, 7.27; S, 5.65.

Example 6

4'-Nitrobenzyl 7-phenoxyacetamido-3-hydroxy-3-cephem-4-carboxylate 1-oxide propionic acid solvate

A 20 g portion of impure 4'-nitrobenzyl 7-phenoxyacetamido-3-hydroxy-3-hydroxy-3-cephem-4-carboxylate 1-oxide was dissolved in 50 ml of dimethylformamide (DMF) with warming. The resulting solution was filtered through a glass frit using 10 ml of DMF. Propionic acid (120 ml) was added to the filtrate. Crystallization was initiated by scratching the sides of the flask containing the mixture. Then 60 ml of water was added dropwise as the crystallization progressed. After stirring the mixture for 60 minutes at room temperature the crystalline product was filtered and washed with 2:1 propionic acid water. Vacuum drying at 45—50°C overnight gave 14.76 g of beige colored crystals: m.p. 149—150°C.

nmr (DMSOd—6) $\delta$ .96 (t, 3, $CH_3CH_2COOH$), 2.2 (q, 2, $CH_3CH_2COOH$), 4.0 (bs, 2, $C_2$—H), 4.65 (bs, 2,

$C_6H_5OCH_2$—), 5.0 (d, l, J = 4 Hz, $C_6$—H), 5.45 (bs, 2, ester $CH_2$), 5.86 (q, l, J = 4 = and 10 Hz, $C_7$—H) and 6.7—8.2 (ArH).

Anal. Calc. for $C_{25}H_{25}N_3O_{11}S$:

C, 52.17; H, 4.38; N, 7.30; O, 30.58; S, 5.57.

Found:

C, 52.26; H, 4.26; N, 7.18; O, 30.72; S, 5.55.

**Claims**

1. A cephalosporin solvate of the formula

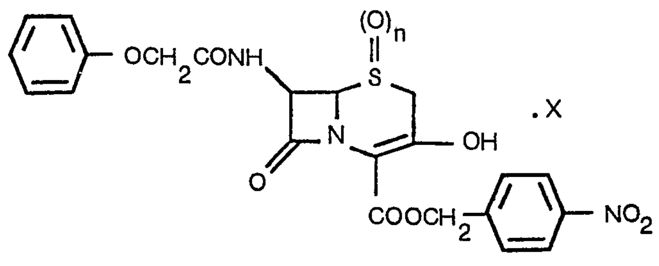

I

wherein n is 1 or 0; and X is $CH_3COOH$, $CH_3CH_2COOH$ or $CH_3OH$ when n is 1, and X is $CH_3COOH$, $CH_3CH_2COOH$ or 1/2 $CH_2Cl_2$ when n is 0.

2. The cephalosporin acetic acid solvate of claim 1 of the formula

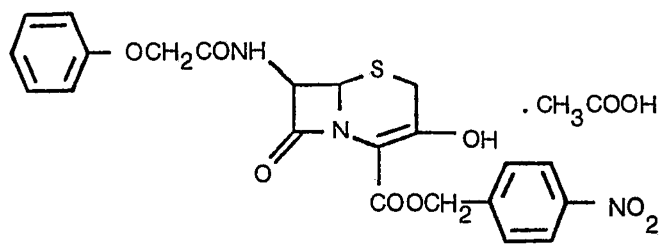

II

3. The cephalosporin sulfoxide acetic acid solvate of claim 1 of the formula

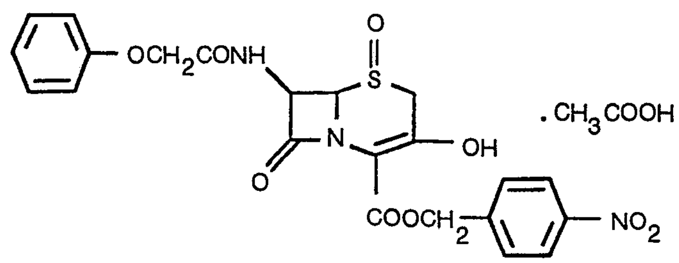

III

4. The cephalosporin methylene chloride solvate of claim 1 of the formula

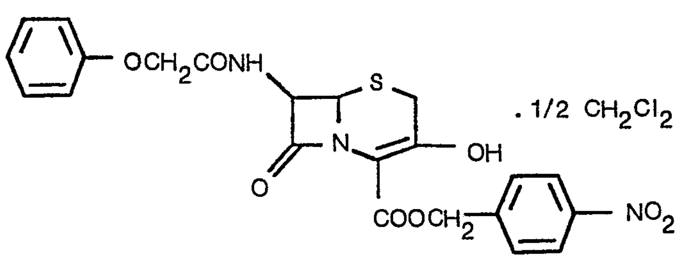

IV

5. The cephalosporin methanol solvate of claim 1 of the formula

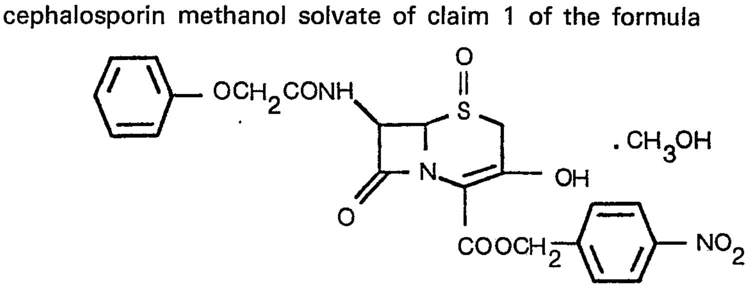

V

6. The cephalosporin propionic acid solvate of claim 1 of the formula

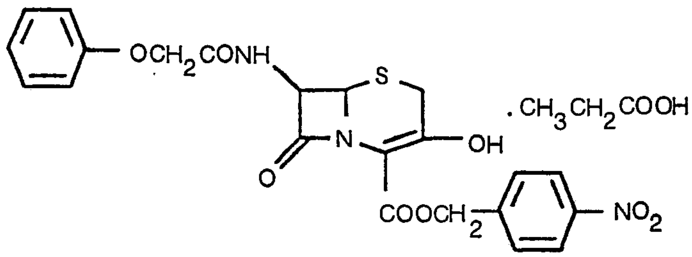

VI

7. The cephalosporin sulfoxide propionic acid solvate of claim 1 of the formula

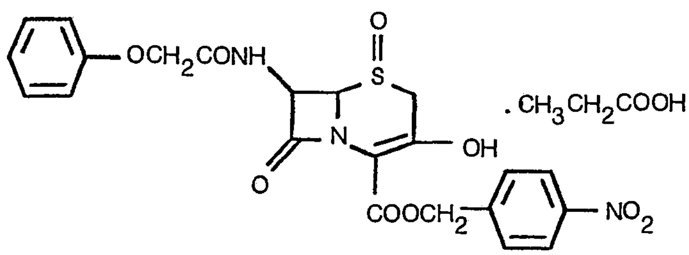

VII

8. A process for preparing a cephalosporin solvate of the formula

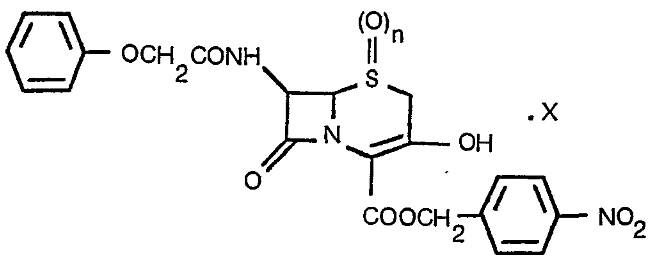

I

wherein n is 1 or 0; and X is $CH_3COOH$, $CH_3CH_2COOH$ or $CH_3OH$ when n is 1, and X is $CH_3COOH$, $CH_3CH_2COOH$ or 1/2 $CH_2Cl_2$ when n is 0 which comprises

a) when n is 1 or 0, contacting 4′-nitrobenzyl 7-phenoxyacetamido-3-hydroxy-3-cephem carboxylate sulfide or sulfoxide thereof with acetic or propionic acid, optionally in the presence of a water miscible or a water immiscible solvent and crystallizing an acetic or propionic solvate therefrom;

b) when n is 0, dissolving the 4′ nitrobenzyl 7-phenoxyacetamido-3-hydroxy-3-cephem carboxylate sulfide in methylene chloride and crystallizing a methylene chloride solvate therefrom.

9. A process as described in claim 8 wherein the sulfide or sulfoxide cephem is dissolved in a water miscible or water immiscible solvent and then acetic or propionic acid is mixed with the solution.

10. A process as described in claim 8 wherein the sulfide or sulfoxide cephem is dissolved in acetic acid and crystallization is effected by adding water to the solution.

11. A process according to claim 8 for preparing the solvate of Formula I wherein X is methanol which comprises slurring an acetic acid or propionic acid solvate according to claim 1, 2 or 3 in methanol and removing the resulting acid-methanol mixture.

**Revendications**

1. Solvate de céphalosporine répondant à la formule:

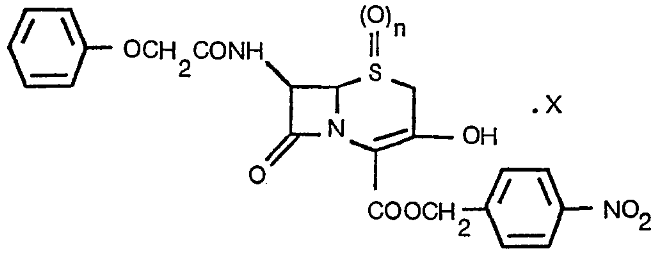

I

dans laquelle n est égal à 1 ou 0; X représente $CH_3COOH$, $CH_3CH_2COOH$ ou $CH_3OH$ lorsque n est égal à 1, et X représente $CH_3COOH$, $CH_3CH_2COOH$ ou $\frac{1}{2}$ $CH_2Cl_2$, lorsque n est égal à 0.

2. Solvate de céphalosporine et de l'acide acétique suivant la revendication 1, répondant à la formule:

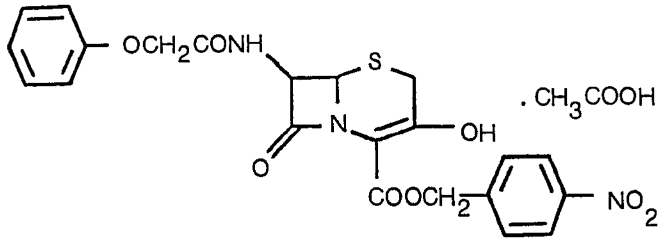

$. CH_3COOH$ II

3. Solvate de sulfoxyde de céphalosporine et d'acide acétique suivant la revendication 1, répondant à la formule

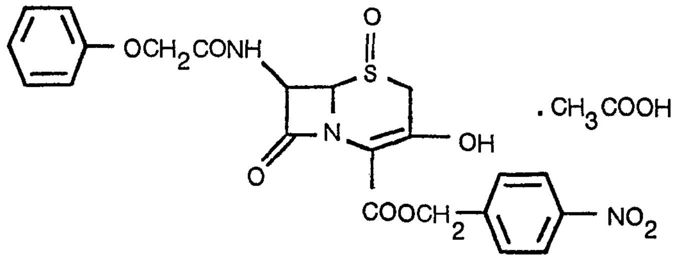

$. CH_3COOH$ III

4. Solvate de céphalosporine et de chlorure de méthylène suivant la revendication 12, répondant à la formule:

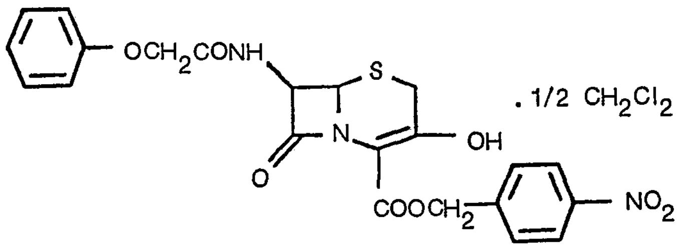

$. 1/2\ CH_2Cl_2$ IV

5. Solvate de céphalosporine et de méthanol suivant la revendication 1, répondant à la formule:

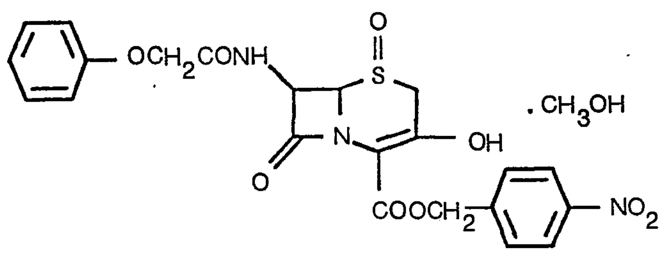

$. CH_3OH$ V

6. Solvate de céphalosporine et de l'acide propionique suivant la revendication 1, répondant à la formule:

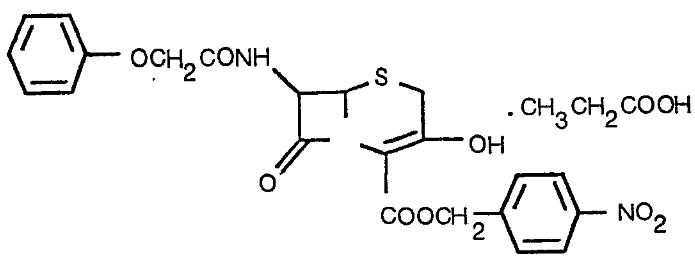

$. CH_3CH_2COOH$ VI

7. Solvate de sulfoxyde de céphalosporine et de l'acide propionique suivant la revendication 1, répondant à la formule:

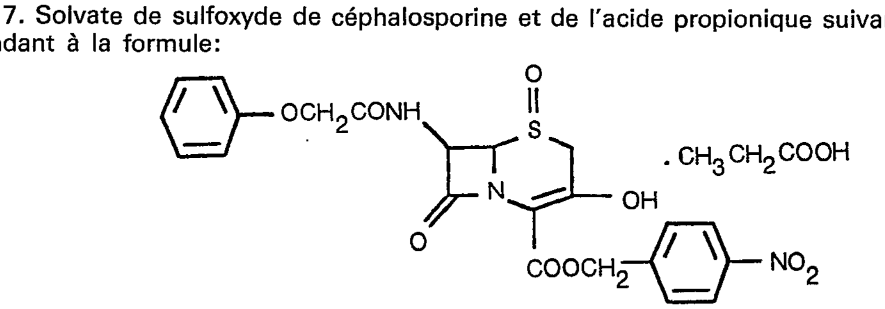

$. CH_3CH_2COOH$ VII

8. Procédé de préparation d'un solvate de céphalosporine de formule:

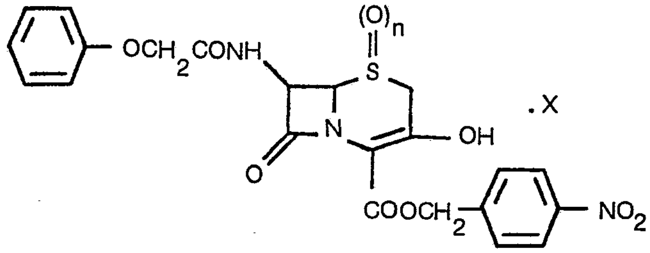

I

dans laquelle n est égal à A ou O; X représente $CH_3COOH$, $CH_3CH_2COOH$ ou $CH_3OH$, lorsque n est égal à 1, et X représente $CH_3COOH$, $CH_3CH_2COOH$ ou $\frac{1}{2}$ $CH_2Cl_2$, lorsque n est égal à 0, procédé caractérisé en ce qu'il consiste

a) lorsque n est égal à 1 ou 0, à mettre du sulfure ou du sulfoxyde de 7-phénoxyacétamido-3-hydroxy-3-céphèm-carboxylate de 4'-nitrobenzyle en contact avec de l'acide acétique ou de l'acide propionique, éventuellement en présence d'un solvant miscible ou non-miscible à l'eau, et à faire cristalliser un solvate acétique ou propionique à partir du mélange susdit;

b) lorsque n est égal à 0, à dissoudre le sulfure de 7-phénoxyacétamido-3-hydroxy-3-céphèm-carboxylate de 4'-nitrobenzyle dans du chlorure de méthylène et à faire cristalliser un solvate de chlorure de méthylène à partir du mélange susdit.

9. Procédé suivant la revendication 8 caractérisé en ce qu'on dissout le sulfure ou le sulfoxyde de céphème dans un solvant miscible ou non-miscible à l'eau et en ce qu'on mélange de l'acide acétique ou de l'acide propionique avec la solution.

10. Procédé suivant la revendication 8 caractérisé en ce qu'on dissout le sulfure ou le sulfoxyde de céphème dans l'acide acétique et en ce qu'on effectue une cristallisation en ajoutant de l'eau à la solution.

11. Procédé suivant la revendication 8 pour préparer le solvate de formule I dans laquelle X représente du méthanol, caractérisé en ce qu'il consiste à mettre un solvate d'acide acétique ou d'acide propionique suivant la revendication 1, 2 ou 3 en boue dans le méthanol et à éliminer le mélange acide/méthanol obtenu.

**Patentansprüche**

1. Cephalosporinsolvate der allgemeinen Formel I

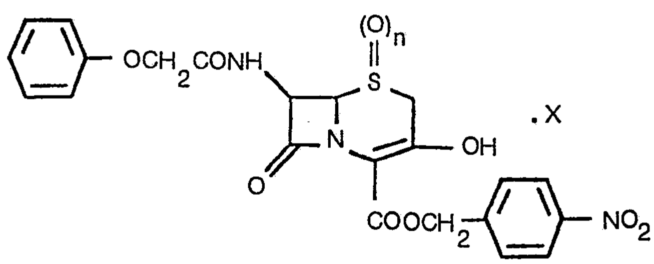

I

worin n für 1 oder 0 steht, wobei X für $CH_3COOH$, $CH_3CH_2COOH$ oder $CH_3OH$ steht, falls n die Zahl 1 ist, und X für $CH_3COOH$, $CH_3CH_2COOH$ oder 1/2 $CH_2Cl_2$ steht, falls n die Zahl 0 bedeutet.

2. Cephalosporinessigsäuresolvat nach Anspruch 1 der Formel II

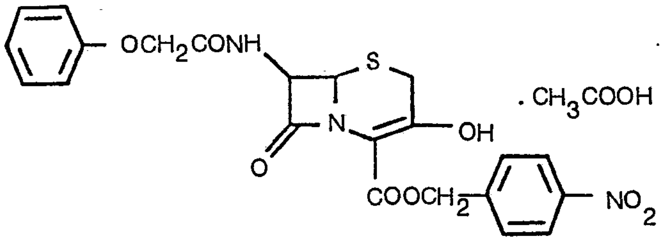

II

3. Cephalosporinsulfoxidessigsäuresolvat nach Anspruch 1 der Formel III

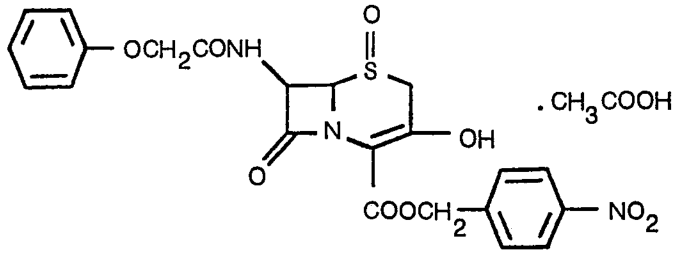

III

4. Cephalosporinmethylenchloridsolvat nach Anspruch 1 der Formel IV

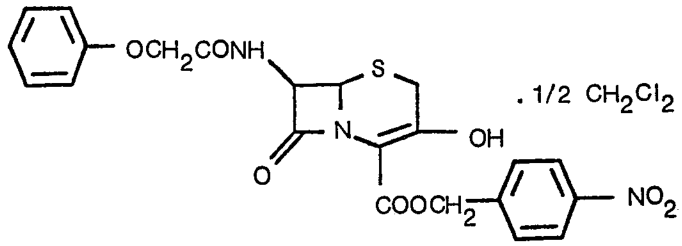

IV

5. Cephalosporinmethanolsolvat nach Anspruch 1 der Formel V

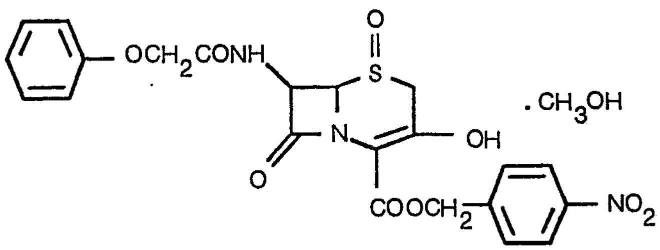

V

6. Cephalosporinpropionsäuresolvat nach Anspruch 1 der Formel VI

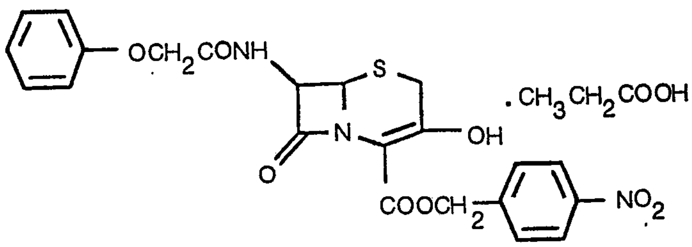

VI

7. Cephalosporinsulfoxidpropionsäuresolvat nach Anspruch 1 der Formel VII

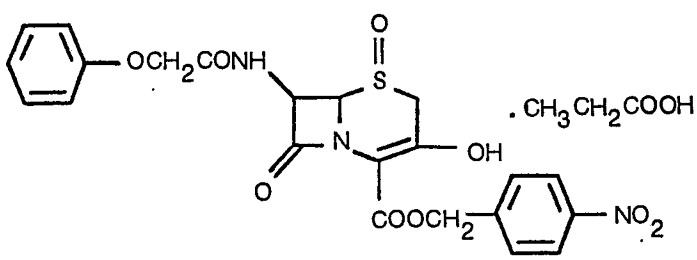

VII

8. Verfahren zur Herstellung eines Cephalosporinsolvats der Formel I

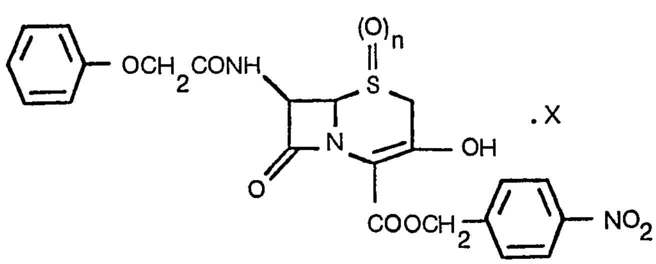

I

worin n für 1 oder 0 steht, wobei X für $CH_3COOH$, $CH_3CH_2COOH$ oder $CH_3OH$ steht, falls n die Zahl 1 ist, und X für $CH_3COOH$, $CH_3CH_2COOH$ oder 1/2 $CH_2Cl_2$ steht, falls n die Zahl 0 bedeutet, dadurch gekennzeichnet, daß man

a) falls n für 0 oder 1 steht, ein 4'-Nitrobenzyl-7-phenoxyacetamido-3-hydroxy-3-cephem-carboxylat-sulfid oder -sulfoxid mit Essigsäure oder Propionsäure, gegebenenfalls in Gegenwart eines mit Wasser mischbaren oder nicht mit Wasser mischbaren Lösungsmittels, umsetzt und daraus dann ein Essigsäuresolvat oder Propionsäuresolvat kristallisiert, oder

b) falls n für 0 steht, das 4'-Nitrobenzyl-7-phenoxyacetamido-3-hydroxy-3-cephemcarboxylat-sulfid in Methylenchlorid löst und daraus dann ein Methylenchloridsolvat kristallisiert.

9. Verfahren nach Anspruch 8, dadurch gekennzeichnet daß man das Sulfidcephem oder Sulfoxidcephem in einem mit Wasser mischbaren oder mit Wasser nicht mischbaren Lösungsmittel löst und mit der dabei erhaltenen Lösung dann Essigsäure oder Propionsäure vermischt.

10. Verfahren nach Anspruch 8, dadurch gekennzeichnet, daß man das Sulfidcephem oder Sulfoxidcephem in Essigsäure löst und die Lösung zur Krsitallisation mit Wasser versetzt.

11. Verfahren nach Anspuch 8 zur Herstellung des Solvats der Formel I, worin X Methanol ist, dadurch gekennzeichnet, daß man ein Essigsäuresolvat oder Propionsäuresolvat nach Anspruch 1, 2 oder 3 in Methanol aufschlämmt und das dabei erhaltene Säure-Methanol Gemisch entfernt.